(19) Europäisches Patentamt
European Patent Office
Office européen des brevets

(11) Veröffentlichungsnummer: **0 238 850 B1**

# (12) EUROPÄISCHE PATENTSCHRIFT

(45) Veröffentlichungstag der Patentschrift: **06.12.89**

(51) Int. Cl.⁴: **C07C 43/11**, C07C 41/16

(21) Anmeldenummer: **87102368.5**

(22) Anmeldetag: **19.02.87**

(54) **Verfahren zur Herstellung von Diethylenglykoldialkylethern.**

(30) Priorität: **26.02.86 DE 3606190**

(43) Veröffentlichungstag der Anmeldung: **30.09.87 Patentblatt 87/40**

(45) Bekanntmachung des Hinweises auf die Patenterteilung: **06.12.89 Patentblatt 89/49**

(84) Benannte Vertragsstaaten: **BE CH DE FR GB IT LI NL**

(56) Entgegenhaltungen:
**SYNTHESIS, Nr. 2, Februar 1979, Seiten 123,124, Georg Thieme Publishers; BORIS G. ZUPANCIC et al.: "Zur Glykol-Veretherung im Fest-Flüssig-Zweiphasensystem"**

(73) Patentinhaber: **HOECHST AKTIENGESELLSCHAFT, Postfach 80 03 20, D-6230 Frankfurt am Main 80(DE)**

(72) Erfinder: **Schwenk, Ulrich, Dr., Robert-Koch-Strasse 209, D-8263 Burghausen/Salzach(DE)**
Erfinder: **Streitberger, Horst, Josef-Hofmiller-Weg 9, D-8262 Altötting(DE)**
Erfinder: **Schulz, Hildegard, Graf-Lehnberger-Strasse 2, D-8343 Triftern(DE)**

EP 0 238 850 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Dialkylethern des Diethylenglykols.

Zur Herstellung dieses Diglykoldialkylethers sind bereits verschiedene Verfahren bekannt.

Nach der klassischen Methode wird er dadurch hergestellt, daß man zunächst den Diglykolmonoalkylether bereitet, davon das Alkalimetallalkoholat herstellt und dieses mit einem Alkylhalogenid umsetzt.

Nach einer anderen Methode erhält man den in Rede stehenden Diether durch Umsetzung von einem Dialkylether, beispielsweise Dimethylether, mit Ethylenoxid in Gegenwart spezieller Katalysatoren. Dabei werden Ethylenoxid-Einheiten zwischen einer Alkylgruppe und dem Sauerstoffatom des Dialkylethers eingeschoben. Bei dieser Umsetzung entsteht unter anderem eine mehr oder weniger große Menge an höheren Glykolethern, die oft unerwünscht sind.

Schließlich ist es aus "Synthesis - Communications", Februar 1979, Seite 123, bekannt, Polyethylenglykoldimethylether dadurch herzustellen, daß man das Polyglykol mit pulverisiertem Alkalimetallhydroxid und Alkylhalogenid umsetzt. Ausgehend von Triethylenglykol, Ätznatron und Chlormethyl wird beispeilsweise Triethylenglykoldimethylether in einer Ausbeute von 75 % erhalten. Im Falle von Diethylenglykol als zu verethernde Verbindung wird jedoch eine Ausbeute von nur 3 % erreicht.

Nun ist aber gerade Diethylenglykol ein wohlfeiles großtechnisches Produkt, das zudem in großer Reinheit erhältlich ist.

Die Aufgabe der Erfindung besteht darin, ein einfaches Verfahren zur beidseitigen Veretherung von Diethylenglykol zur Verfügung zu stellen, bei dem keine weiteren Ether wie solche von höheren Homologen, gebildet werden.

Das erfindungsgemäße Verfahren zur Herstellung von Dialkylethern des Diethylenglykols ist dadurch gekennzeichnet, daß man Diethylenglykol in Gegenwart eines Lösungsmittels, ausgewählt aus der Gruppe der höheren Ethylenglykoldialkylether, mit mindestens 2 mol Alkalimetallhydroxid und mindestens 2 mol Alkylhalogenid pro mol Diglykol umsetzt und aus dem Reaktionsprodukt den Diethylenglykoldialkylether gewinnt.

Es war überraschend, daß die Veretherung des in Rede stehenden Diglykols mit einem Alkylhalogenid und mit Alkalimetallhydroxid bei Verwendung von höheren Ethylenglykoldialkylethern als Lösungsmittel (Verdünnungsmittel) erreicht werden kann, und dies in nahezu quantitativer Ausbeute.

Beim erfindungsgemäßen Verfahren wird von Diethylenglykol ($HOCH_2CH_2OCH_2CH_2OH$) als zu verethernder Verbindung ausgegangen.

Das Diethylenglykol wird zur Überführung in den entsprechenden Dialkylether in Gegenwart eines Lösungsmittels mit einem Alkylhalogenid und mit Alkalimetallhydroxid umgesetzt. Als Lösungsmittel (Verdünnungsmittel) werden erfindungsgemäß höhere Ethylenglykoldialkylether eingesetzt. Diese Polyglykolether entsprechen der Formel:

$RO(CH_2CH_2O)_xR'$.

In der Formel ist R und R′ bevorzugt die Methyl-, Ethyl-, Propyl- oder eine Butylgruppe und besonders bevorzugt die Methyl- oder Ethyl-Gruppe. R und R′ sind bevorzugt gleich. x ist eine Zahl von 3 bis 20, vorzugsweise 4 bis 10, insbesondere 4 bis 7. Als Lösungsmittel kann erfindungsgemäß einer dieser Ether eingesetzt werden, in der Regel wird es sich um eine Mischung mehrerer solcher Ether handeln; so sind im Handel derartige Ethermischungen mit beispielsweise x = 4 bis 6, 4 bis 8 oder 5 bis 10 erhältlich. Die Menge (Gewichtsmenge) an Lösungsmittel beträgt zweckmäßigerweise das 1,5- bis 5fache, vorzugsweise das 1,5- bis 3fache, bezogen auf die Gewichtsmenge an eingesetztem Diethylenglykol.

Als Alkylhalogenid wird im allgemeinen ein Methyl, Ethyl-, Propyl- oder ein Butylhalogenid eingesetzt. Ebenso wie die Alkylgruppe ist auch das Halogenatom im Alkylhalogenid nicht kritisch. Die Alkylgruppe ist vorzugsweise Methyl oder Ethyl und das Halogenatom ist vorzugsweise Chlor oder Iod. Das Alkylhalogenid wird in der bei Normalbedingungen vorliegenden Form in einer Menge von mindestens 2 mol, vorzugsweise in einer Menge von 2,1 bis 3 mol, pro mol Diethylenglykol, eingesetzt. Größere Mengen als 3 mol sind wegen des relativ hohen Partialdruckes des Alkylhalogenids bei der während der Umsetzung herrschenden Temperatur in der Regel nicht zweckmäßig.

Als Alkalimetallhydroxid wird vorzugsweise das Natrium- oder Kaliumhydroxid eingesetzt. Es wird in fester (gekörnter oder pulverisierter) Form in einer Menge von mindestens 2 mol, vorzugsweise in einer Menge von 2,5 bis 4 mol, pro mol Diethylenglykol, eingesetzt. Größere Mengen als 5 mol wären zwar reaktionskinetisch in der Regel vorteilhaft, derart große Mengen an Alkalimetallhydroxid erschweren jedoch die technische Reaktionsführung.

Die Umsetzung nach dem erfindungsgemäßen Verfahren ist exotherm und wird bei einer Temperatur von 30 bis 100 °C, vorzugsweise 40 bis 80 °C durchgeführt. Aufgrund der Umsetzungstemperatur und der Reaktionsteilnehmer, insbesondere des Alkylhalogenids, herrscht während der Umsetzung ein Druck von bis zu etwa 6 bar. Das Ende der Umsetzung wird durch den abgefallenen Druck angezeigt.

Aus dem nach dem erfindungsgemäßen Verfahren erhaltenen Reaktionsprodukt wird der angestrebte Diethylenglkyoldialkylether vorzugsweise auf destillativem Wege gewonnen.

Nachstehend wird eine bevorzugte Durchführung des erfindungsgemäßen Verfahrens im einzelnen beschrieben:

Das zu verethernde Diethylenglykol, pulverisiertes oder gekörntes Alkalimetallhydroxid und das Lösungsmittel werden in einem Reaktionsgefäß vorgelegt und durch Rühren gut vermischt. Nach Evakuieren des Reaktionsgefäßes, beispielsweise mit einem Wasserstrahlvakuum oder nach Spülen mit Stickstoff zur Entfernung der Luft aus dem Reaktionsgefäß, wird die Mischung aus den vorgelegten

Substanzen vorzugsweise auf 40 bis 80 °C erhitzt und unter Beibehaltung dieser Temperatur das Alkylhalogenid zudosiert; es wird also derart zudosiert, daß die exotherme Reaktion innerhalb des angegebenen bevorzugten Temperaturbereiches bleibt. Im Reaktionsgefäß herrscht der insbesondere vom Alkylhalogenid resultierende Druck. Nachdem das einzusetzende Alkylhalogenid zudosiert und umgesetzt ist, was am abgefallenen und konstantbleibenden Druck ersichtlich ist, wird aus dem erhaltenen Reaktionsprodukt der angestrebte Diethylenglykoldialkylether gewonnen. Dazu wird vorzugsweise so vorgegangen, daß zunächst die Festprodukte, das ist das gebildete Alkalimetallhalogenid und das nicht-umgesetzte Alkalimetallhydroxid, abfiltriert werden und aus dem Flüssigprodukt der angestrebte Diethylenglykoldialkylether destillativ gewonnen wird, oder daß zunächst das Reaktionsprodukt mit Wasser versetzt wird, um zwei etwa gleich große Phasen (nämlich eine wäßrige und eine organische Phase) zu erhalten, worauf aus der abgetrennten organischen Phase der angestrebte Diethylenglykoldialkylether destillativ gewonnen wird. Das jeweils zurückgewonnene Lösungsmittel kann beim nächsten Ansatz wieder eingesetzt werden.

Während der Umsetzung bei 50 °C kann die Aufnahme von Chlormethyl trotz überschüssigen Alkalis zum Stillstand kommen. Der Grund für dieses Verhalten ist eine Viskositätserhöhung, die das Reagieren von gasförmigem Chlormethyl und der Flüssigphase praktisch verhindert. Durch Aufwärmen auf 80 °C wird die Viskositätserhöhung abgebaut und die Reaktion, die zum Stillstand gekommen war, läßt sich ohne Schwierigkeiten bis auf mehr als 99 % Umsatz weiterführen, auch bei 40 °C.
Beim erfindungsgemäßen Verfahren können im Prinzip auch Mischungen von Diethylenglykol mit höheren Ethylenglykolen, das sind Polyethylenglykole wie Triethylenglykol und dergleichen, oder mit höheren Ethylenglykolmonoalkylethern, das sind Alkylpolyethylenglykole wie Methylpolyglykol 250, eingesetzt werden. So kann man zum Beispiel die Methylierung von Methylpolyglykol 250 mit der Bismethylierung von Diglykol kombinieren und somit aus Methylpolyglykol und Diglykol, Dimethylpolyglykol und Dimethyldiglykol herstellen.

Das erfindungsgemäße Verfahren weist eine Reihe von Vorteilen auf. So ist das bei der Umsetzung gebildete Kochsalz von hoher Reinheit und kann nach Auswaschen mit Wasser in einer Elektrolyse, vorzugsweise einer Diaphragma-, Amalgam- oder Ionenmembranelektrolyse eingesetzt werden, gegebenenfalls nach Neutralisation mit Salzsäure. Bei dem neuen Verfahren kommt es zu keinen Wärmestaureaktionen, wie sie beispielsweise auftreten, wenn man Diglykol mit einer stöchiometrischen Menge Ätznatron-Pulver bei höheren Temperaturen zusammenbringt. Das erfindungsgemäße Verfahren erfordert relativ niedrige Reaktionstemperaturen, und es wird trotzdem ein quantitativer Umsatz zu einer Hydroxylzahl von etwa 1 erreicht; dies ist ein völlig unerwartetes Ergebnis. Die bei der Umsetzung entstehende wäßrige konzentrierte Natronlauge wird vom auskristallisierten Kochsalz praktisch vollständig aufgenommen. Will man das Kochsalz als gesättigte Lösung gewinnen, so gibt man zum Reaktionsprodukt soviel Wasser hinzu, daß alles Salz bei 30 °C gelöst ist, und erwärmt dann auf 90 °C; es scheidet sich eine obere, organische Phase ab und eine gesättigte wäßrige Kochsalzlösung als untere Phase (eventuell darin vorhandenes Lösungsmittel scheidet sich beim Eindampfen der Kochsalzlösung ab).

Die Erfindung wird nun an Beispielen noch näher erläutert:

<u>Beispiel 1</u>

In einem 1-Liter-Glasautoklaven wurden 159 g (1,5 mol) Diethylenglykol technischer Qualität, 156 g (3,9 mol) Ätznatron-Perlen und 300 ml Tetraethylenglykoldimethylether als Verdünner vorgelegt und unter Rühren vermischt, worauf der Glasautoklav zur Entfernung der Luft mit Stickstoff gespült und anschließend kurz unter Wasser strahlvakuum gesetzt wurde. Nun wurde der Inhalt des Glasautoklaven auf 40 °C erhitzt und nach Erreichen dieser Temperatur wurde mit der Zudosierung von (gasförmigem) Methylchlorid begonnen. Innerhalb von 10 h wurden insgesamt 200 ml (3,7 mol) Methylchlorid zudosiert, wobei der Druck im Glasautoklaven auf etwa 2 bar und die Temperatur nach anfangs 40 °C auf 60 °C anstieg. Bei dieser Temperatur wurde die Methylierung zu Ende geführt (ersichtlich am abgefallenen und konstantgebliebenen Druck). Zur Aufarbeitung des Reaktionsproduktes nach Entspannen des Autoklaven wurde zunächst das gebildete Natriumchlorid und das nicht-umgesetzte Ätznatron abfiltriert und anschließend das Filtrat mit Hilfe einer 30 cm langen Spiegelglaskolonne bei einem Rücklaufverhältnis von 1 : 5 fraktioniert destilliert.
Es wurden 176,6 g Diethylenglykoldimethylether erhalten, das ist eine Ausbeute von 89 Gew.-% der Theorie.

<u>Beispiel 2</u>

In einem 1-Liter-Glasautoklaven wurden 159 g (1,5 mol) Diethylenglykol technischer Qualität, 156 g (3,9 mol) Ätznatron-Perlen und 300 ml Dimethylpolyglykol 250 als Verdünner vorgelegt und unter Rühren vermischt, worauf der Glasautoklav zur Entfernung der Luft mit Wasserstrahlvakuum evakuiert wurde. Nun wurde der Inhalt des Glasautoklaven auf 40 °C erhitzt und nach Erreichen dieser Temperatur wurde mit der Zudosierung von (gasförmigem) Methylchlorid begonnen. Innerhalb von 15 h wurden insgesamt 200 ml (3,7 mol) Methylchlorid zudosiert, wobei der Druck im Glasautoklaven auf etwa 2 bar und die Temperatur nach anfangs 40 °C auf 60 °C anstieg. Bei dieser Temperatur wurde die Methylierung zu Ende geführt (ersichtlich am abgefallenen und konstantgebliebenen Druck). Zur Aufarbeitung des Reaktionsproduktes nach Entspannen des Glasautoklaven wurden zum Reaktionsprodukt 560 ml Wasser dazugegeben, worauf sich nach einem 15 min langen Rühren bei 80 °C zwei gut voneinander getrennte Schichten bildeten, nämlich eine obere organische Phase und eine untere wäßrige Phase. Nach Abtrennung der organischen Phase wurde diese wie in Beispiel 1 fraktioniert destilliert.

Es wurden 177,0 g Diethylenglykoldimethylether erhalten, das ist eine Ausbeute von 88 Gew.-% der Theorie.

**Patentansprüche**

1. Verfahren zur Herstellung von Dialkylethern des Diethylenglykols, dadurch gekennzeichnet, daß man Diethylenglykol in Gegenwart eines Lösungsmittels, ausgewählt aus der Gruppe der höheren Ethylenglykoldialkylether mit mindestens 2 mol Alkalimetallhydroxid und mindestens 2 mol Alkylhalogenid pro mol Diglykol umsetzt und aus dem Reaktionsprodukt den Diethylenglykoldialkylether gewinnt.

2. Verfahren nach Anspruch 1, dadurch gekennzeichnet, daß als Lösungsmittel ein Ethylenglykoldialkylether der allgemeinen Formel $RO(CH_2CH_2O)_xR'$ eingesetzt wird, worin R und R' eine Methyl-, Ethyl-, Propyl- oder eine Butylgruppe und x eine Zahl von 3 bis 20 ist.

3. Verfahren nach Anspruch 1, dadurch gekennzeichnet, daß als Lösungsmittel ein Tetra-, Penta-, Hexa- oder Heptaethylenglykoldimethyl- oder -diethylether oder eine Mischung von zwei oder mehreren dieser Ether eingesetzt wird.

4. Verfahren nach einem oder mehreren der Ansprüche 1 bis 3, dadurch gekennzeichnet, daß als Alkalimetallhydroxid Natriumhydroxid oder Kaliumhydroxid eingesetzt wird.

5. Verfahren nach einem oder mehreren der Ansprüche 1 bis 4, dadurch gekennzeichnet, daß als Alkylhalogenid Methylchlorid oder Ethylchlorid eingesetzt wird.

6. Verfahren nach einem oder mehreren der Ansprüche 1 bis 5, dadurch gekennzeichnet, daß die Umsetzung bei einer Temperatur von 40 bis 80 °C durchgeführt wird.

**Claims**

1. A process for the preparation of diethylene glycol dialkyl ethers characterised by reacting diethylene glycol in the presence of a solvent, selected from the group of the higher ethylene glycol dialkyl ethers, with at least 2 moles of an alkali metal hydroxide and at least 2 moles of an alkyl halogenide per mole of diethylene glycol, and recovering the diethylene glycol dialkyl ether from the reaction product.

2. A process as claimed in claim 1, wherein an ethylene glycol dialkyl ether of the general formula $RO(CH_2CH_2O)_xR'$, wherein R and R' represent a methyl, ethyl, propyl or a butyl group and x is a number of from 3 to 20, is used as the solvent.

3. A process as claimed in claim 1, wherein a tetra-, penta-, hexa- or heptaethylene glycol dimethyl- or diethylether or a mixture of two or more of these ethers are used as solvent.

4. A process as claimed in one or more of the claims 1 to 3, wherein sodium hydroxide or potassium hydroxide are used as the alkali metal hydroxide.

5. A process as claimed in one or more of the claims 1 to 4, wherein methyl chloride or ethyl chloride are used as the alkyl halogenide.

6. A process as claimed in one or more of the claims 1 to 5, wherein the reaction is carried out at a temperature of from 40 to 80°C.

**Revendications**

1. Procédé pour préparer des éthers dialkyliques du diéthylène-glycol, procédé caractérisé en ce qu'on fait réagir le diéthylène-glycol en présence d'un solvant pris dans l'ensemble constitué par les éthers dialkyliques d'éthylène-glycols supérieurs, avec au moins 2 mol d'un hydroxyde de métal alcalin et au moins 2 mol d'un halogénure d'alkyle par mole du diglycol, et on isole du produit réactionnel l'éther dialkylique du diéthylène-glycol.

2. Procédé selon la revendication 1 caractérisé en ce qu'on utilise comme solvant un éther dialkylique de l'éthylène-glycol qui répond à la formule générale: $RO(CH_2CH_2O)_xR'$ dans laquelle R et R' représentent chacun un radical méthyle, éthyle, propyle ou butyle et x désigne un nombre de 3 à 20.

3. Procédé selon la revendication 1 caractérisé en ce qu'on utilise comme solvant un éther diméthylique ou diéthylique du tétra-éthylène-glycol, du penta-éthylèneglycol, de l'hexa-éthylène-glycol ou de l'hepta-éthylène-glycol, ou un mélange de deux ou de plus de deux de ces éthers.

4. Procédé selon l'une quelconque des revendications 1 à 3, caractérisé en ce qu'on utilise, comme hydroxyde de métal alcalin, l'hydroxyde de sodium ou l'hydroxyde de potassium.

5. Procédé selon l'une quelconque des revendications 1 à 4, caractérisé en ce qu'on utilise, comme halcgénure d'alkyle, le chlorure de méthyle ou le chlorure d'éthyle.

6. Procédé selon l'une quelconque des revendications 1 à 5, caractérisé en ce qu'on effectue la réaction à une température de 40 à 80°C.